(19) **Europäisches Patentamt**

**European Patent Office**

**Office européen des brevets**

(11) Numéro de publication: **0 301 954**
**A2**

(12) # DEMANDE DE BREVET EUROPEEN

(21) Numéro de dépôt: **88401935.7**

(22) Date de dépôt: **25.07.88**

(51) Int. Cl.⁴: **C 12 N 15/00**
C 12 N 9/88, C 12 P 21/02

(30) Priorité: **24.07.87 FR 8710614**

(43) Date de publication de la demande:
**01.02.89 Bulletin 89/05**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15 (FR)**

(72) Inventeur: **Danchin, Antoine**
**60 rue de Reuilly**
**F-75012 Paris (FR)**

**Glaser, Philippe**
**28 rue Chapon**
**F-75003 Paris (FR)**

**Ullmann, Agnès**
**3 rue Paul Dupuy**
**F-75016 Paris (FR)**

(74) Mandataire: **Peaucelle, Chantal et al**
**S.C. Ernest Gutmann - Yves Plasseraud 67, boulevard Haussmann**
**F-75008 Paris (FR)**

Le (Les) microorganisme(s) a (ont) été déposé(s) auprès Collection Nationale de Cultures de Microorganismes sous le(s) numéro(s) I-678.

(54) **Procédé de clonage et d'expression de gènes codant pour des protéines constituant des édifices fonctionnels organisés.**

(57) Le procédé de l'invention comprend la transformation de cellules d'une souche de microorganisme réceptrice en introduisant des fragments d'ADN constitués par, ou comprenant, au moins deux gènes étrangers interchangeables, et capables d'exprimer dans les cellules réceptrices, des protéines constituant des édifices fonctionnels organisés.

Le gène indicateur est capable d'exprimer une protéine permettant de détecter l'activité biologique de la protéine exprimée par le gène à révéler, cette activité résultant de l'interaction entre les protéines. L'activité biologique de la protéine recherchée est déétectée par la mise en oeuvre d'un moyen physicochimique.

Ce procédé est avantageusement appliqué à la production par exemple d'adénylate cyclase ou de calmoduline.

EP 0 301 954 A2

## Description

# PROCEDE DE CLONAGE ET D'EXPRESSION DE GENES CODANT POUR DES PROTEINES CONSTITUANT DES EDIFICES FONCTIONNELS ORGANISES

L'invention a pour objet un procédé de clonage et d'expression de gènes codant pour des protéines constituant des édifices fonctionnels organisés.

Un très grand nombre de gènes actifs codent pour des protéines dont les activités s'exercent, ou sont inhibées, à la suite d'interactions avec les protéines spécifiées par d'autres gènes. Ces systèmes multiprotéiques sont le plus souvent des systèmes homologues c'est-à-dire dérivés d'un même organisme. Ils peuvent être parfois hétérologues et sont formés de protéines interagissant entre elles, mais provenant d'organismes différents.

Les principales méthodes de clonage connues à ce jour permettent l'intégration d'une seule unité étrangère ou d'une suite contigue d'unités, que l'on détectera après clonage. Cependant, elles ne sont pas utilisées pour le clonage de plusieurs unités génétiques ayant une parenté fonctionnelle, mais qui peuvent n'avoir aucune parenté, ni par leur position d'origine dans un chromosone, ni même de parenté d'organisme d'origine.

Il existe bien des dispositifs variés mettant simultanément sur une unité réplicative des gènes d'origines diverses; c'est le cas par exemple d'un gène cloné, considéré en même temps qu'un gène de résistance à un antibiotique sur un plasmide de clonage. Mais il s'agit là d'une association contingente, qui n'implique aucune parenté fonctionnelle entre les gènes étudiés. Il existe aussi des opérons qui représentent une suite fonctionnelle préexistante, présente dans un organisme donné. On a également proposé d'associer deux gènes différents, clonés sur une même unité réplicative, l'un étant un gène de contrôle, qui permet l'expression de l'autre. Grâce à cette association, il est possible d'obtenir une expression conditionnelle du gène cloné et de s'affranchir ainsi de certaines contraintes de toxicité. En général, il s'agit du clonage simultané d'un répresseur, contrôlable par un paramètre physique comme la température, ou par la présence d'un inducteur que l'on peut ajouter à volonté au milieu. Dans ce cas, l'association des gènes ne correspond aucunement à une parenté d'activité du produit des gènes clonés simultanément.

En outre, il est à noter que dans les procédés de clonage des gènes, il est généralement nécessaire d'obtenir une quantité suffisante du produit exprimé pour pouvoir le révéler.

Il peut être de plus difficile ou impossible de cloner le gène lorsque le produit exprimé se révèle délétère.

L'invention a pour but de remédier au moins en partie à ces difficultés et d'améliorer les techniques proposées à ce jour permettant le clonage direct des gènes ou l'expression simultanée des systèmes multiprotéiques.

L'invention vise plus spécialement à fournir un procédé permettant de produire simultanément une série de protéines distinctes, selon une association fonctionnelle qui n'existe pas en tant qu'organisation génétique préexistante dans le ou les organismes considérés.

Le procédé de clonage et d'expression de l'invention est caractérisé en ce que :
- on transforme les cellules d'une souche de microorganisme réceptrice, c'est-à-dire capable d'accueillir de l'ADN étranger et de l'exprimer, en introduisant des fragments d'ADN constitués par, ou comprenant, au moins deux gènes étrangers, à savoir au moins un gène indicateur et au moins un gène à révéler, ces gènes étant, le cas échéant, interchangeables en tant que gènes indicateurs ou gènes à révéler, et capables d'exprimer dans les cellules réceptrices, des protéines constituant des édifices fonctionnels organisés, le gène indicateur étant capable d'exprimer une protéine permettant de détecter l'activité biologique de la protéine exprimée par le gène à révéler, cette activité biologique résultant de l'interaction entre les protéines,
- on détecte l'activité biologique de la protéine recherchée par mise en oeuvre par exemple d'un moyen physico-chimique, biochimique, microbiologique ou radioimmunologique et, dans les systèmes homologues, et le cas échéant, dans les systèmes hétérologues,
- on isole le gène codant pour cette protéine en mettant en jeu une propriété caractéristique du gène.

On observera qu'on constitue, avec ce procédé, un ensemble fonctionnel artificiel au sein d'un organisme permettant l'expression.

Le gène indicateur et le gène révélateur ayant un role symétrique, le clonage et l'expression peuvent concerner des protéines à sous-unités multiples dont l'activité biologique n'est présente que lorsque plusieurs sous-unités sont simultanément exprimées.

Selon un aspect de l'invention, le procédé défini ci-dessus fournit des moyens pour isoler un gène codant pour une sous-unité protéique donnée d'un ensemble fonctionnel, à partir de différents fragments d'ADN susceptibles de le renfermer.

L'invention permet ainsi la caractérisation moléculaire du gène, et donc son expression.

Selon un autre aspect, l'invention fournit des moyens de production en masse des protéines exprimées par les gènes révélés, ou à la fois par les gènes indicateurs et les gènes révélés, ou encore des protéines résultant de l'interaction des protéines exprimées par les gènes étrangers introduits, et ce, de manière avantageuse, chez divers organismes cellulaires récepteurs.

Les produits tels qu'obtenus par le procédé de l'invention entrent également dans le cadre de cette dernière. En particulier, l'invention vise les séquences nucléotidiques clonées et les protéines exprimées, celles-ci étant caractérisées par leurs propriétés de coopération entre elles.

De nombreuses souches réceptrices conviennent pour la mise en oeuvre de l'invention, dès lors qu'elles sont capables d'accueillir de l'ADN étranger et de l'exprimer. Il s'agit en particulier de souches, fonctionnant selon le code génétique universel.

On citera les souches bactériennes, les levures, les cellules eucaryotes.

De préférence, les souches bactériennes utilisées présentent un caractère de restriction moins, ce qui permet une mise en oeuvre directe du procédé de l'invention, c'est-à-dire sans passage préalable par une souche intermédiaire restriction moins.

Pour permettre la détection de la production d'une protéine donnée, ces souches ont avantageusement une déficience stable, et non réversible, à un taux élevé, dans les gènes spécifiant la synthèse de protéines ayant une activité homologue à celle de la protéine ou des protéines à exprimer.

Une illustration comprend le clonage et l'expression de protéines multimériques eucaryotes dans une bactérie où il n'y a pas de contrepartie homologue. Il s'agit par exemple du cas de l'expression d'immunoglobulines fonctionnelles ou d'hémoglobines chez une bactérie.

Selon une variante de l'invention, les gènes introduits sont des gènes impliqués dans des systèmes hétérologues. Cette variante sera développée plus loin dans le cas du clonage et de l'expression d'une toxine bactérienne dont l'activité dépend de la présence d'une protéine eucaryote et dans celui du clonage de cette protéine eucaryote.

Conformément à l'invention, on effectue de préférence un clonage successif des gènes codant pour les diverses unités de l'édifice multiprotéique.

Selon ce mode de réalisation, on introduit successivement dans la cellule réceptrice des fragments d'ADN, de préférence portés par des réplicons différents, comportant respectivement les gènes indicateurs et les gènes à révéler.

Ces réplicons sont de préférence compatibles entre eux.

Pour permettre un dosage de l'expression, on utilise de préférence un contrôle s'exerçant simultanément sur plusieurs gènes. Il peut s'agir par exemple de l'association entre un plasmide porteur du répresseur thermosensible du bactériophage lambda (pDIA 9205) et d'un plasmide de groupe d'incompatibilité différent porteur d'un promoteur contrôlé par ce répresseur (pDIA 3237 voir référence (1) à la fin de la description). Un contrôle de ce type, efficace chez E.coli, permet avantageusement l'expression de protéines dimériques, comme les immunoglobulines.

D'une manière avantageuse, l'invention permet ainsi l'expression de protéines eucaryotes multimériques dans une bactérie.

En variante, la transformation de la cellule réceptrice est réalisée en n'utilisant qu'un seul type de réplicon, les clonages ultérieurs sont effectués sur le réplicon en un site permettant la conservation fonctionnelle du premier gène introduit.

Les réplicons sont avantageusement choisis parmi les plasmides, cosmides et bactériophages utilisables de manière habituelle comme vecteurs. Le chromosome de la cellule réceptrice peut être également utilisé.

Des plasmides appropriés pour un clonage chez E.coli comprennent les dérivés du réplicon colE1 (groupe Inc. Q), pACYC184 (groupe Inc. P15A) ou pDIA13 (groupe Inc W)(1).

La détection de l'activité biologique résultant de l'interaction entre les protéines exprimées dans la cellule réceptrice est avantageusement réalisée par un moyen physico-chimique, biochimique (enzymatique), microbiologique (test de croissance). On citera encore le dosage de la production d'un métabolite par technique spectroscopique, étude de l'apparition d'une activité biologique par croissance sur milieux appropriés.

Le gène codant pour la protéine recherchée est isolé en mettant en jeu une propriété caractéristique du gène, telle qu'une résistance à un antibiotique ou à un microorganisme virulent, ou encore l'aptitude à croître sur un milieu dans lequel la souche originale ne peut croître, ou toute autre révélation microbiologique.

Une autre application importante du procédé de l'invention consiste dans le clonage de gènes codant pour des protéines permettant d'élaborer, par exemple à la suite d'interactions avec d'autres protéines, des produits toxiques utilisables, notamment, comme principes vaccinants. La production en grande quantité de ces protéines dans des microorganismes revêt également un grand intérêt.

Cet aspect de l'invention sera illustré par le clonage du gène de B.pertussis codant pour l'adénylate cyclase et la production de la protéine toxique.

On connaît la pathogénicité élevée de B.pertussis, bactérie responsable de la coqueluche. Parmi les éléments impliqués dans le caractère pathogène de cette bactérie, il semble qu'un phénomène majeur soit la production considérable d'AMP cyclique (ou AMPc) par les cellules infectées. Cette production fait intervenir un mécanisme d'activation de l'adénylate cyclase produite par B.pertussis, par une protéine de l'hôte, la calmoduline.

Il s'agit donc dans ce cas d'un système protéique formé de deux sous-unités, la calmoduline et l'adénylate cyclase, spécifiées chacune par des gènes d'organismes différents.

L'utilisation conformément à l'invention, de l'interaction entre ces deux sous-unités, permet l'isolement du gène codant pour l'adénylate cyclase, dont les difficultés de clonage par les méthodes habituelles sont, par ailleurs, bien connues.

Selon la stratégie de l'invention, on introduit dans une cellule un réplicon A, contenant le gène indicateur codant pour la calmoduline, puis on introduit dans cette cellule les fragments de l'ADN total de B.pertussis dans un réplicon B.

Les réplicons A et B sont avantageusement choisis parmi ceux définis ci-dessus et la cellule réceptrice est plus spécialement une cellule bactérienne, telle que E.coli. On y distingue un site catalytique, le site de liaison à la calmoduline. Ensuite est située une portion responsable de l'activité hémolytique de la protéine.

Il va de soi, cependant, qu'un seul vecteur de clonage peut également être utilisé : le réplicon A porteur d'un gène indicateur, dans lequel on clonera par criblage le gène à révéler.

Par criblage des fragments de l'ADN total de B.pertussis, il est possible d'isoler le gène codant pour l'adénylate cyclase, en révélant par exemple à l'aide d'un test coloré sur milieu de maltose, la production d'AMPc qui résulte de l'activation de l'adénylate cyclase par la calmoduline, dans une souche réceptrice dépourvue d'adénylate cyclase.

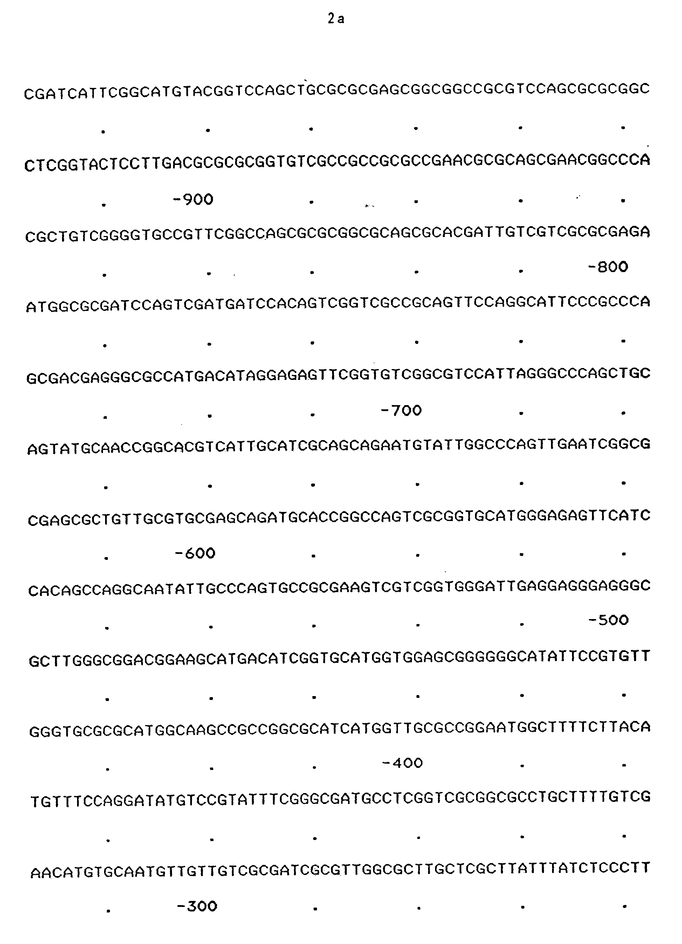

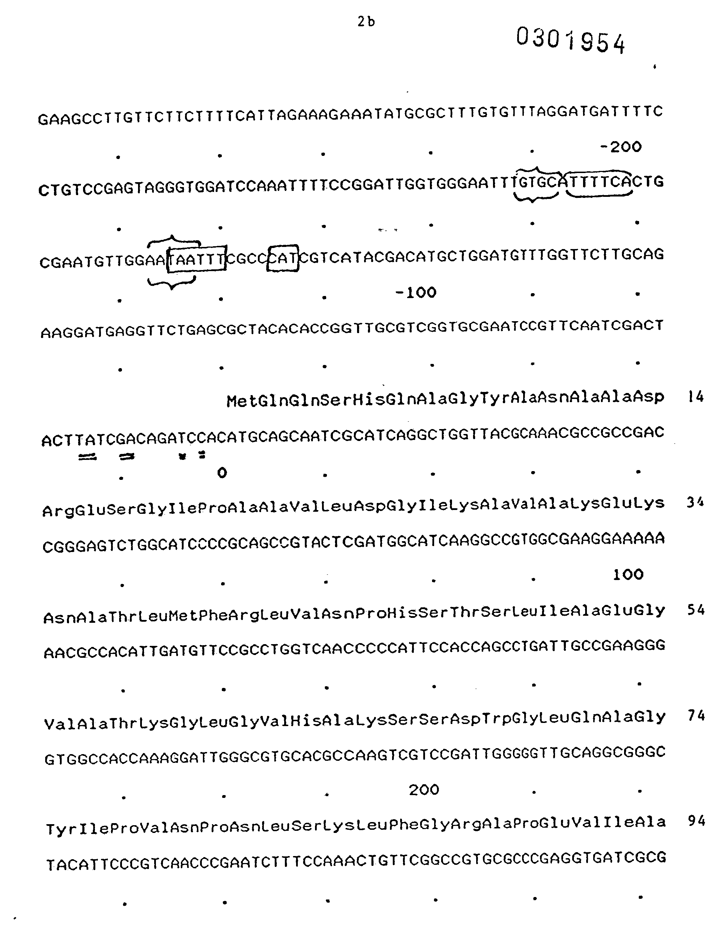

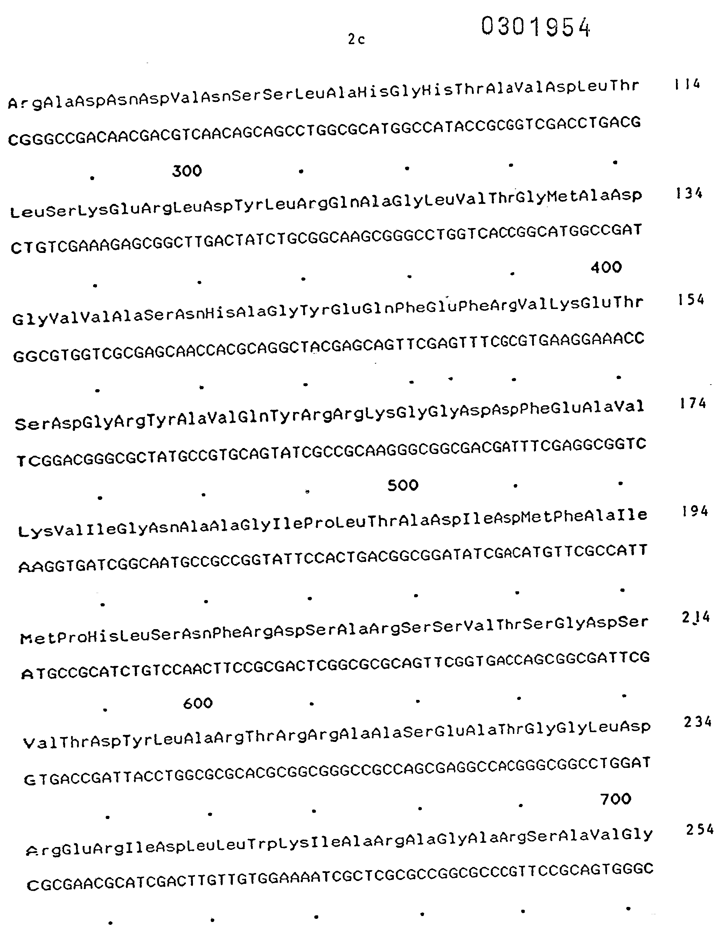

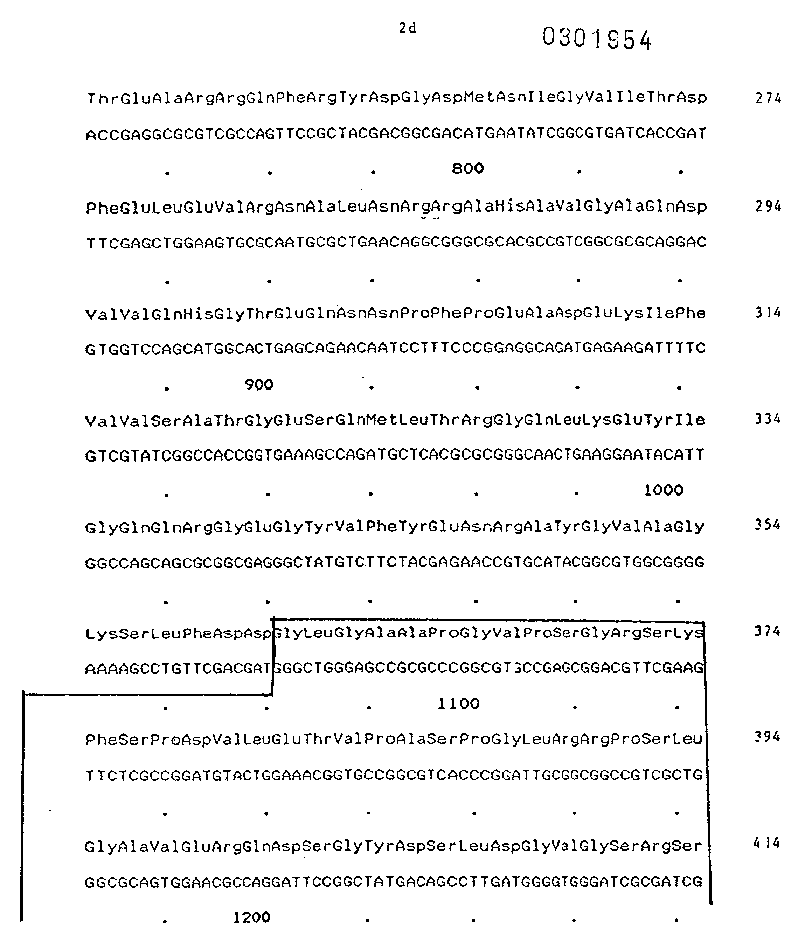

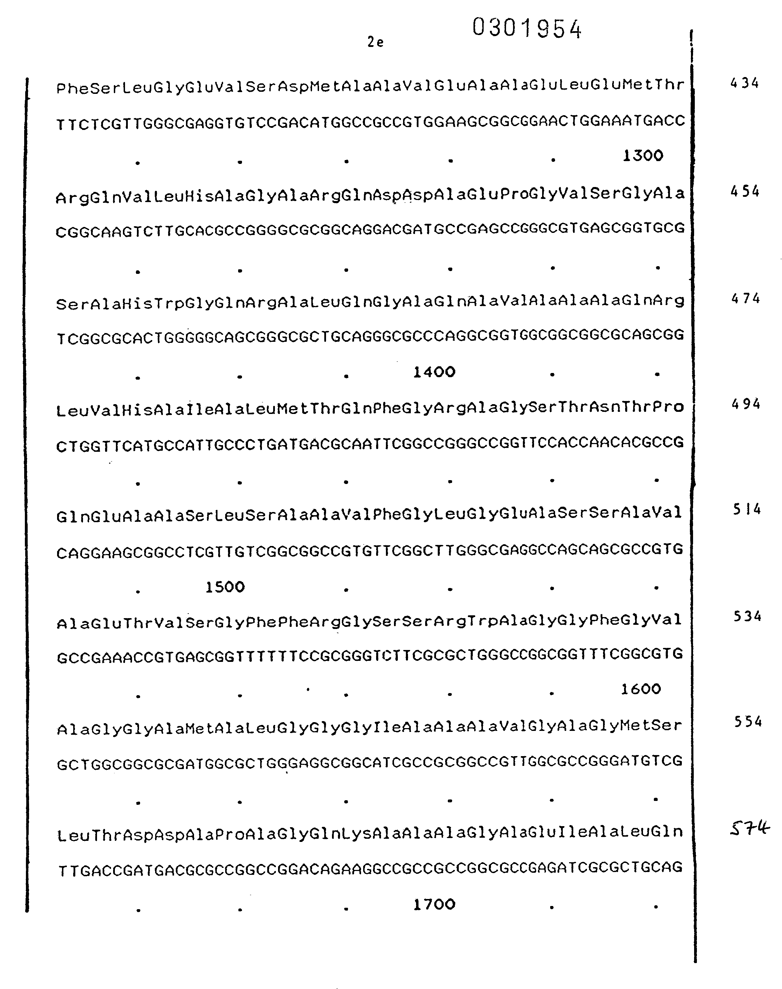

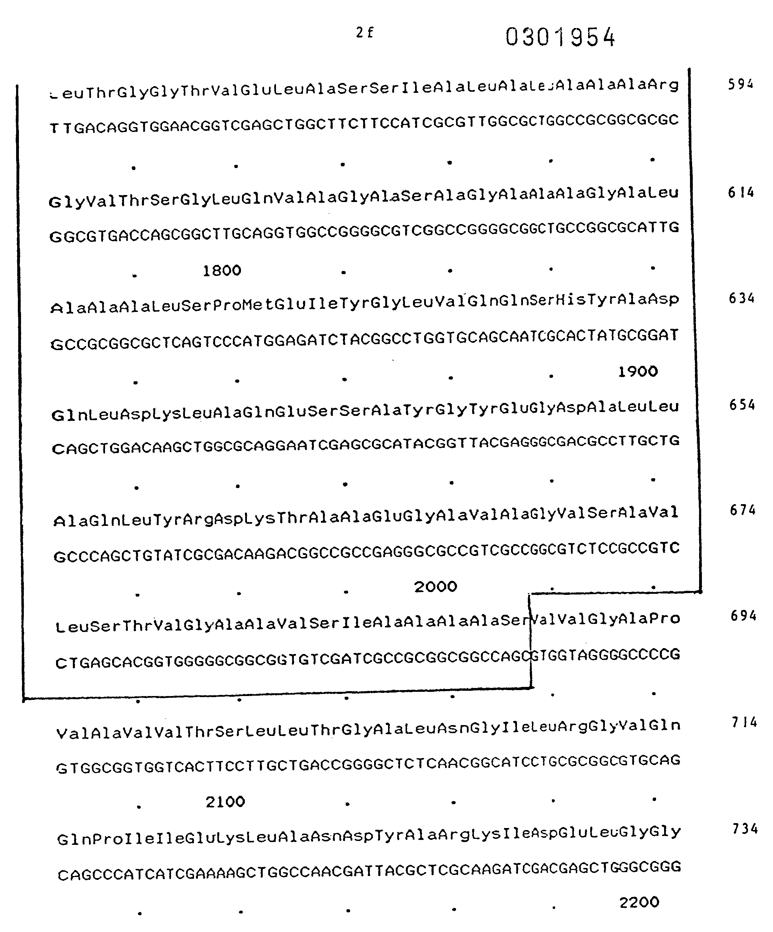

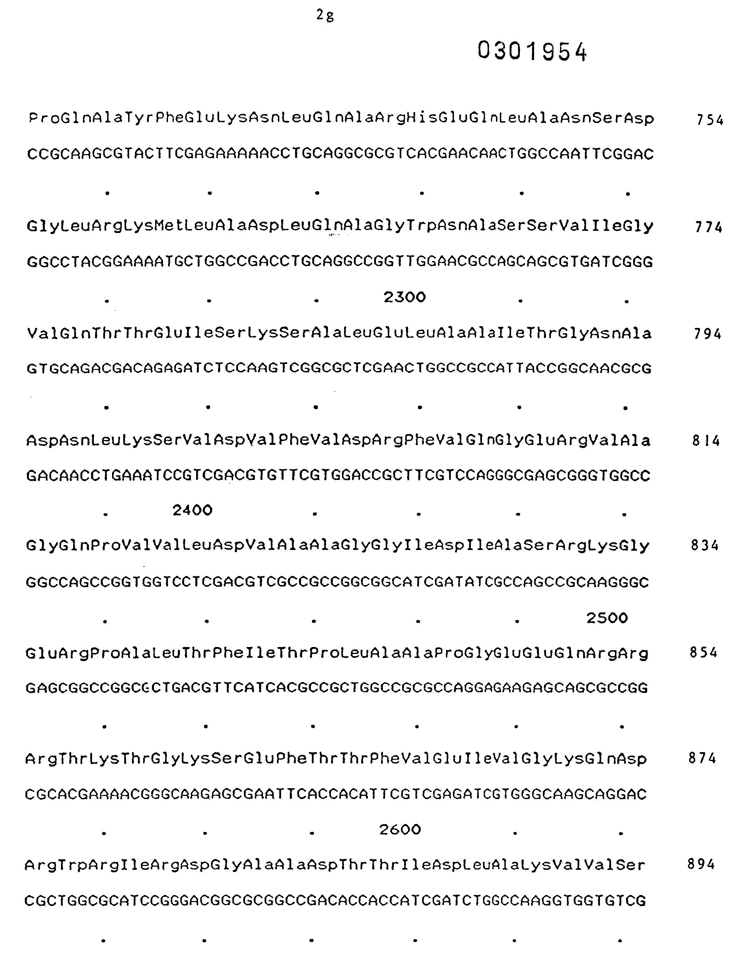

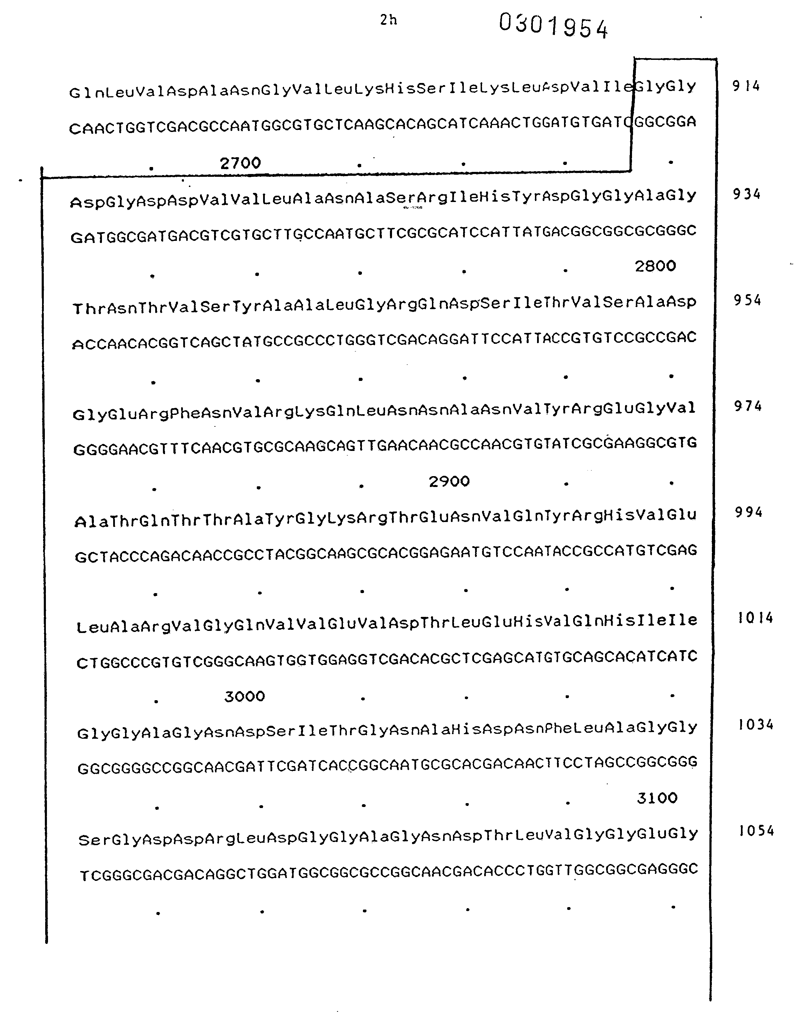

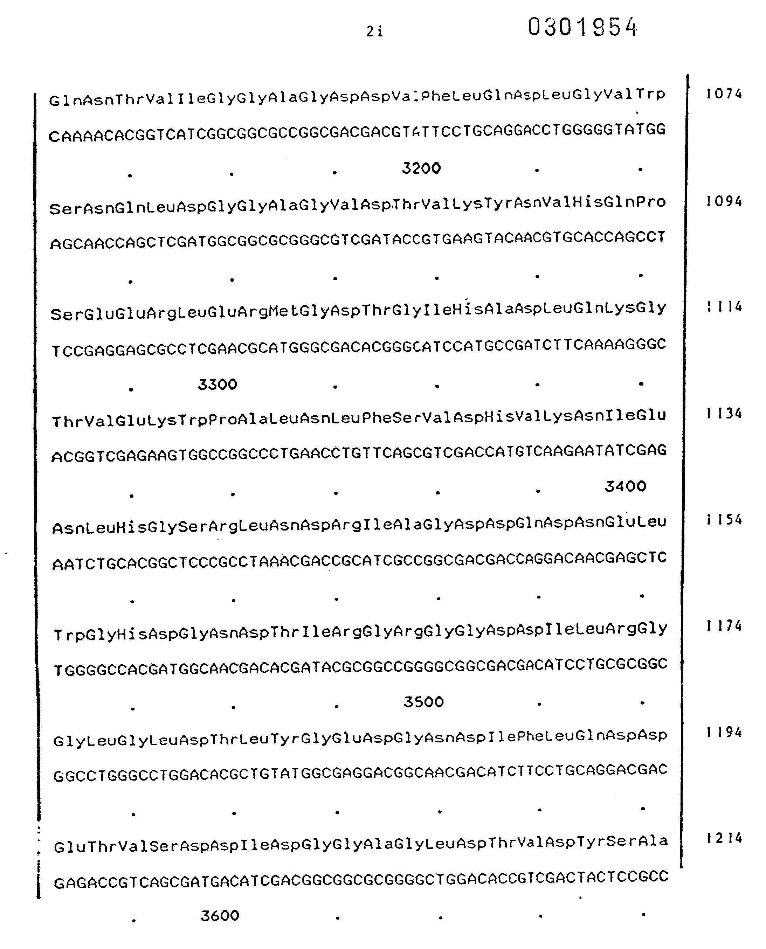

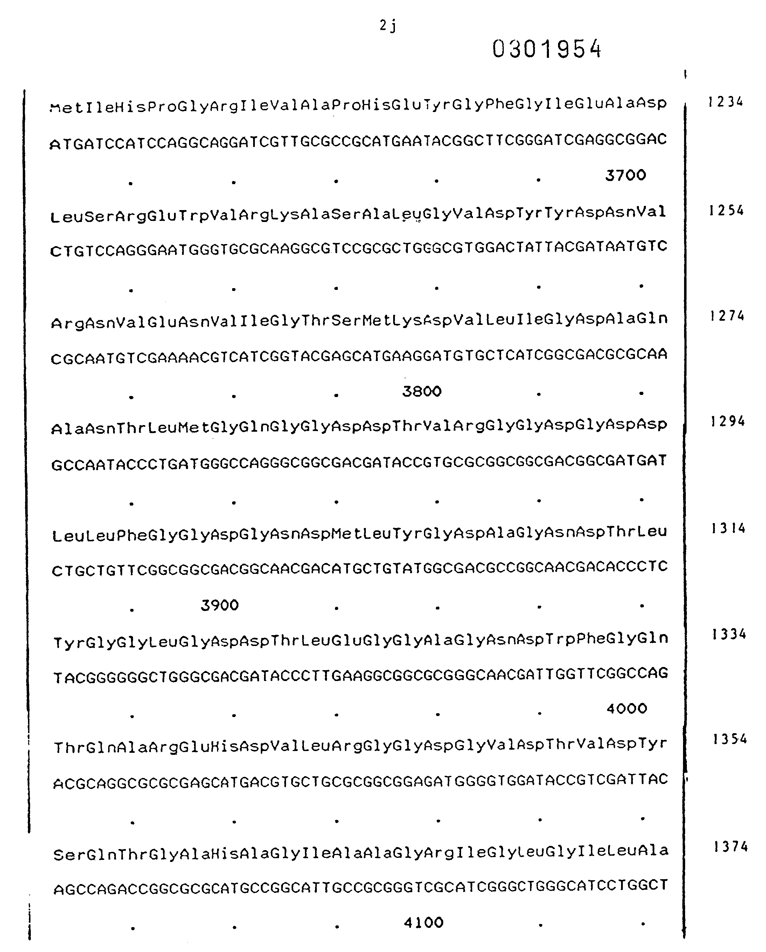

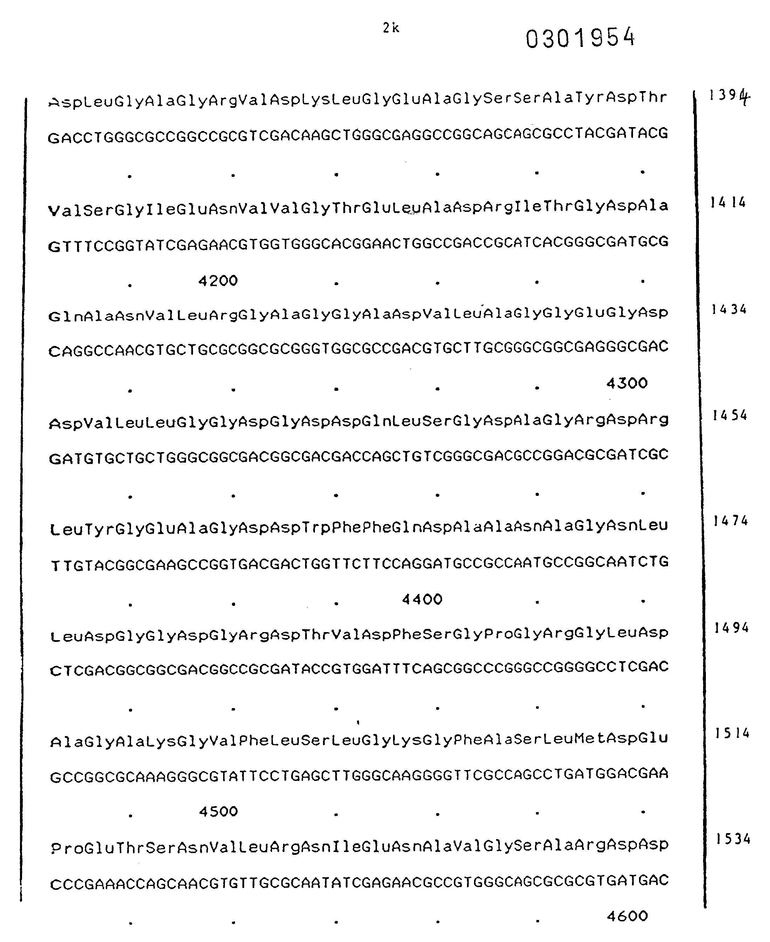

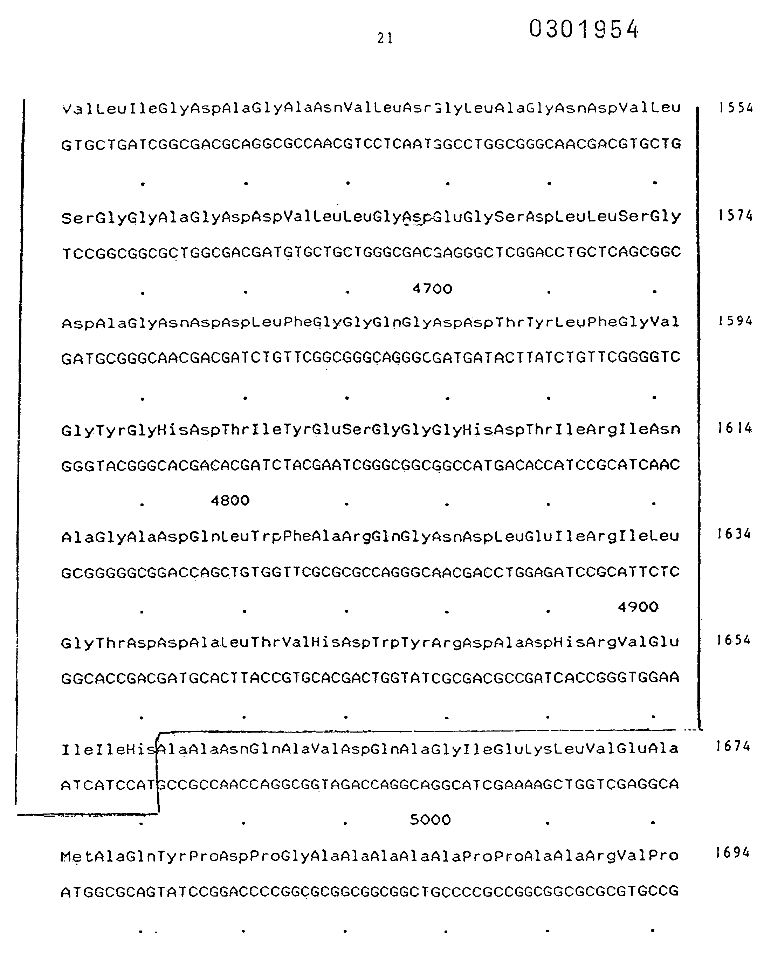

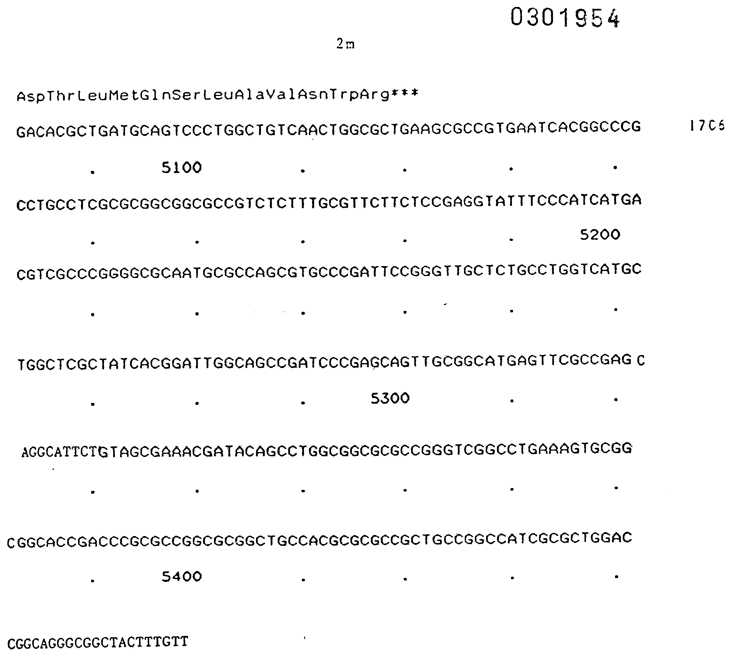

L'invention vise également le fragment d'ADN cloné associé à l'activité hémolytique du gène de l'adénylate cyclase dont la séquence est donnée sur la figure 2. La partie catalytique du fragment d'ADN comprend la séquence de nucléotides située entre les positions 0 et 2 050, la position 0 correspondant au site de restriction BamHI. On y distingue un site catalytique, le site de liaison à la calmoduline. Ensuite est située une portion responsable de l'activité hémolytique de la protéine.

Il va de soi que les bases de la séquence nucléotidique considérée peuvent être dans un ordre différent de celui trouvé dans les gènes et/ou que ces bases peuvent être le cas échéant substituées, dès lors qu'une sonde élaborée à partir d'une telle séquence donne une réponse caractéristique et non équivoque quant à la capacité de reconnaître la présence d'un gène codant pour une protéine à activité adénylate cyclase.

Toute séquence nucléotidique hybridable avec celle de l'enchaînement de la séquence de la figure 2, telle qu'obtenue par transcription enzymatique inverse de l'ARN correspondant ou encore par synthèse chimique entrent également dans le cadre de l'invention.

L'invention concerne également une séquence de nucléotides codant pour la séquence d'acides aminés représentée sur la figure 2a à 2f.

Elle concerne également tout ou partie de cette séquence d'acides aminés et d'une manière générale une protéine à activité adénylate cyclase capable de former un complexe immunologique avec des anticorps dirigés contre des protéines à activité adénylate cyclase.

Entrent également dans le cadre de l'invention les anticorps polyclonaux formés contre la protéine de l'invention ainsi que les anticorps monoclonaux capables de reconnaître spécifiquement cette protéine.

Le procédé de clonage et d'expression de l'invention, qui est basé sur la coopération des protéines codées par des gènes de différentes origines, permet également d'isoler des gènes de la calmoduline d'organismes variés et de réaliser l'activation in vivo de l'adénylate cyclase de B. pertussis produite dans une cellule hôte, en particulier une cellule bactérienne.

Cet aspect de l'invention sera illustré par le clonage du gène de la calmoduline de cerveau de souris.

On introduit dans une cellule réceptrice n'exprimant pas d'adénylate cyclase, un réplicon A1 contenant les fragments d'ADNc de cerveau, puis dans un réplicon B1, le gène de l'adénylate cyclase de B.pertussis. Par criblage, on isole le gène codant pour une protéine capable d'activer l'adénylate cyclase produite par B. pertussis.

D'autres caractéristiques et avantages de l'invention apparaîtront dans les exemples qui suivent et en se reportant aux figures 1 à 2 :

- la figure 1 représentant la carte de restriction de la partie active du gène de l'adénylate cyclase de B. pertussis cloné chez E. coli et

- la figure 2a à 2e, la séquence du fragment d'ADN de la partie du gène correspondant au domaine catalytique, et la séquence codée, correspondante d'acides aminés.

<u>EXEMPLE 1 : Clonage du gène codant pour l'adénylate cyclase de B.pertussis</u>

1/ - Construction de trois souches cya⁻, restriction⁻ de E.coli, à savoir :

- une souche de référence, restriction moins, qui résiste jusqu'à 5 mM d'AMP cyclique (ou AMPc) exogène, et qui permet de détecter aisément 0,5 mM d'AMPc exogène;

- une souche dite bas niveau, restriction moins, qui permet de détecter 50 µM d'AMPc exogène, mais dont la sens bilité à l'AMPc apparaît au-dessus de 1 mM;

- une souche dite résistante qui permet de détecter 3 mM d'AMPc et qui résiste à 30 mM au moins de ce nucléotide.

Ces souches sont aisément transformables (à $10^6$ au moins transformants pour 1 µg de pBR322), par la technique classique décrite dans (2).

Ces souches sont avantageusement stabilisées pour être cultivables sur plusieurs générations.

La détection du caractère "production d'AMP cyclique" est réalisée au moyen de tests colorés indiquant la fermentation de certaines sources de carbone comme le maltose. Le caractère cya⁻ peut être aisément sélectionné en mettant à profit la résistance connue de tels mutants à des antibiotiques comme le Mecillinam, ou la phosphomycine et au bactériophage lambda virulent :

100 microlitres d'une culture saturée de la souche initiale sont étalés en présence d'un nombre dix fois plus élevé d'un bactériophage lambda virulent, sur une boîte de Pétri contenant un milieu Mac Conkey Maltose (3), supplémenté avec la concentration léthale minimum pour E.coli de l'antibiotique. Cet antibiotique, en effet, permet une sélection efficace des mutants cya⁻ et des mutants crp⁻, c'est-à-dire respectivement dépourvus d'adénylate cyclase (cya) ou de récepteur de l'AMP cyclique (crp); par ailleurs, les souches cya ou crp sont résistantes au phage lambda virulent et comme elles ne fermentent pas le maltose, elles apparaissent sous la forme de colonies blanches sur le milieu Mac Conkey.

Il convient de s'assurer que les mutants sont effectivement de type cya en confirmant qu'ils retrouvent d'une part leur capacité de croître sur maltose en présence d'AMPc et, d'autre part, qu'ils peuvent croître sur glucose (ce qui exclut certains mutants du système des phosphotransférases (système dépendant du phosphoéno pyruvate : pts)).

Pour obtenir des mutants dits à bas niveau, on utilise 100 µl d'une culture d'un mutant cya préalablement isolé, que l'on fait croître sur un milieu synthétique M63 (3) en présence de 0,4% de maltose et de 50 μM d'AMPc. Les colonies trouvées sont alors réisolées 3 fois sur le même milieu, puis testées pour leur incapacité à croître sur le même milieu dépourvu d'AMPc. On confirme, par ailleurs, leur sensibilité à l'AMPc qui est généralement supérieure à celle de la souche originale.

Pour obtenir des mutants dits à haut niveau, on procède par étapes successives en augmentant, sur un milieu LB (3) la concentration d'AMPc. Parmi les mutants obtenus, un grand nombre sont des mutants crp, qui sont devenus insensibles à l'AMPc, mais qui ne permettent plus de détecter sa présence : il convient donc, à chaque étape (deux étapes, 12 mM et 30 mM, sont généralement suffisantes) de s'assurer que les bactéries forment des colonies blanches en l'absence d'AMPc et rouges en présence de 3 mM d'AMPc sur le milieu Mc Conkey maltose. Les bactéries obtenues sont généralement légèrement dépendantes de la présence d'AMPc et il convient de les faire croître dans les précultures en présence d'une concentration au moins égale à 3 mM d'AMPc, afin de conserver leur caractère résistant de façon stable.

2/ - Transformation des souches

Ces souches sont alors transformées par un plasmide porteur d'un gène spécifiant la synthèse de calmoduline tel que le plasmide pVUC-1, du groupe d'incompatibilité ColE1 et porteur du gène de résistance à l'ampicilline.

On introduit ensuite dans les trois souches des vecteurs renfermant de l'ADN de B.pertussis total fragmenté de diverses manières (coupures partielles par des enzymes de restriction EcoRI et SauIIIa, sonication su vie d'addition de "linkers" EcoRI par exemple).

En raison des problèmes posés par l'incompatibilité d'autres plasmides ayant le réplicon ColEI (voir plus haut) le clonage de fragments d'ADN total de B.pertussis partiellement coupé par l'enzyme SauIIIa a été effectué dans le vecteur compatible pACYC184, au site unique BamH1. Les souches réceptrices ont été transformées et étalées sur des boîtes Mc Conkey Maltose. Plusieurs clones fermentant le maltose ont été isolés. Il a ensuite été démontré, que ces clones produisent de l'AMP cyclique. Par ailleurs, les plasmides extraits des souches productrices ont été intégrés par transformation dans des souches qui soit ne contiennent pas le plasmide pVUC-1, soit le contiennent. Dans le premier cas, les clones sont blancs sur les milieux Mac Conkey maltose, rouges dans le second, confirmant que c'est bien la présence des plasmides permettant la synthèse de calmoduline qui a permis le clonage.

3/ - Détection de l'AMPc

La souche bas niveau a été utilisée pour la détection de la présence d'AMPc de la façon suivante : $10^4$ bactéries bas niveau ont été étalées sur une boîte Mac Conkey Maltose, et une réplication de clones variés, contenant, entre autres, une souche de E.coli crp⁻ et les souches contenant le plasmide pVUC-1 (5) ainsi que les plasmides putatifs contenant le gène de l'adénylate cyclase de B.pertussis, a été effectuée. Les souches ont été mises à incuber à 37°C pendant 20 heures. Puis les colorations des taches observées : les souches cya⁻ donnent des taches blanches, les souches cya⁺ des taches rouges, mais la souche crp⁻ (cya⁺) donne, de plus un halo rouge d'au moins 10mm de diamètre, indiquant que l'AMPc synthétisé dans ce cas, a diffusé et a permis la fermentation du maltose par la souche indicatrice bas niveau. Le même résultat a été obtenu avec les diverses souches contenant le gène putatif de l'adénylate cyclase de B.pertussis en présence du plasmide pVUC-1.

Ce résultat indique une forte production d'AMPc par ces bactéries. Par ailleurs, une confirmation ultérieure a été obtenue par le résultat négatif du catabolisme du maltose dans une souche crp⁻ contenant pVUC-1 et un plasmide porteur du gène cyclase de B.pertussis.

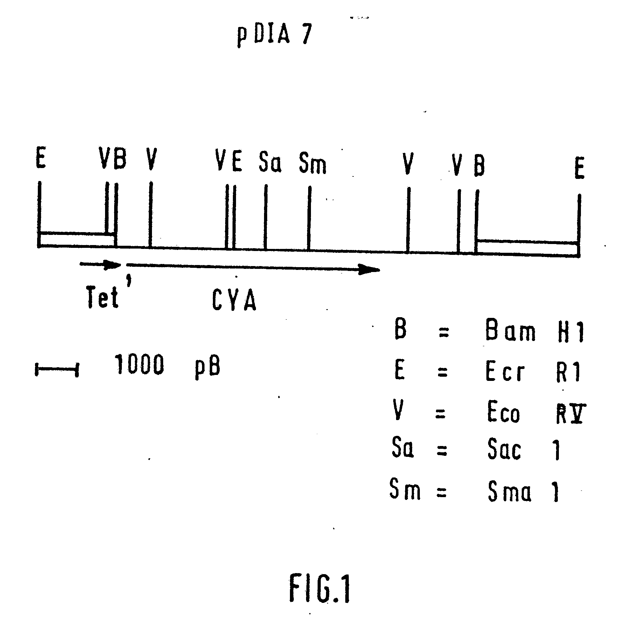

Les caractéristiques du fragment d'ADN contenant la partie active du gène de l'adénylate cyclase de B.pertussis isolée de la souche E. coli Tp610 pDIA7 déposée le 21 juillet 1987 sous le n° I-678 à la CNCM (Collection Nationale de Culture de Microorganismes) sont données sur les figures 1 et 2.

EXEMPLE 2 : Clonage du gène codant pour la calmoduline de cerveau de souris

Souches bactériennes et milieux de croissance

On utilise un dérivé cya de la souche E.coli C600SF8, TP610, restriction⁻ (6). On cultive les bactéries sur un milieu riche LB ou un milieu minimum M63 (3). On effectue un criblage en tenant compte de la capacité à provoquer la fermentation des sucres en utilisant des plaques d'agar Mac Conkey contenant 1% de maltose et de l'expression de la béta-galactosidase. Les concentrations en antibiotiques sont les suivantes : chloramphenicol 30 μg/ml ; ampicilline 100 μg/ml. La souche TP610 est transformée selon Maniatis et al (2) avec le plasmide pDIA5 ce qui la rend incapable de synthétiser AMPc. On utilise cette souche transformée comme hôte pour cloner des fragments d'ADNc de cerveau de souris.

. Construction de banques d'ADNc - Analyse de la séquence d'ADN

Pour analyser les séquences d'ADN, on utilise des sous-clones dans le phage M13tgl31 (7). On engendre des délétions unidirectionnelles en utilisant le système cyclone (IBI). La séquence d'ADN est déterminée par la méthode du didéoxynucléotide (8) en utilisant de l'alpha S$^{35}$ dATP (Amersham) et du 7-déaza-dGTP (Boehringer) à la place de dGTP, ce qui permet une meilleure résolution des régions riches en GC dans les gels de polyacrylamide.

On prépare une banque d'ADNc à amorce oligo(dT) à partir d'ARN poly(A)⁺ de cerveau de souris en utilisant le processus primaire-adapteur de Caput et al (10).

Le plasmide pUC-9 est linéarisé en utilisant PstI et en fixant les extrémités dG à l'aide d'une transférase terminale.

Les molécules obtenues sont digérées par Bam-HI. Le petit fragment engendré par la coupure par BamHI est éliminé par filtration à travers un polysaccharide, notamment de l'agarose tel que celui commercialisé sous la marque Sépharose CL6B.

L'ADNc est produit à partir de 1g d'une matrice d'ARN-m en utilisant une transcriptase inverse avec un oligonucléotide spécifique servant d'amorce.

L'ARN-m restant est éliminé par hydrolyse alcaline et des élongations dC sont effectuées sur l'ADNc en utilisant une terminal transférase. L'extrémité 5′ des ADNc est transformée en une extrémité cohésive BamHI par hybridation avec un adaptateur, à savoir un autre oligonucléotide synthétique complémentaire du primaire. L'ADNc monobrin est alors réuni au vecteur par ligation pour produire des molécules circulaires. On utilise l'extrémité (dG) du vecteur comme amorce et l'ADN T4 polymérase pour réparer la région monobrin.

Avec l'ADN plasmidique recombinant, on transforme des bactéries hautement compétentes (MC 1061, 10⁹ CFU/g d'ADN plasmidique). Les transformants sont sélectionnés en fonction de leur croissance en présence d'ampicilline.

L'un des clones isolés, pDIA70, est purifié et sa séquence d'ADN analysée. A titre de contrôle, on constate que les bactéries produisant pDIA5 ou pDIA70 perdent leur capacité à provoquer la fermentation de maltose ou à exprimer l'activité bêta-galactosidase. Elles retrouvent ces propriétés lorsque pDIA5 et pDIA70 sont présents ensemble dans la même bactérie.

La comparaison avec le gène de calmoduline du rat montre la grande homologie qui existe entre ces deux gènes suggérant que l'ADNc cloné dans le plasmide pDIA70 code par le calmoduline de souris.

La méthode de clonage de l'ADNc de calmoduline de cerveau de souris est également utilisable pour cribler ou sélectionner d'autres systèmes de gènes à composants multiples. En particulier, l'invention fournit ainsi les moyens de détecter des facteurs susceptibles d'interférer avec l'activation par la calmoduline d'activités d'enzymes décelables.

Les exemples décrits ci-dessus illustrent une méthode générale de clonage de gènes et de production dans des cellules réceptrices de leurs produits d'expression et des produits résultant de l'interaction des protéines exprimées.

Ces méthodes s'appliquent, inter alia :
- au clonage du gène de la toxine adénylate cyclase de Bacillus anthracis et à la production de cette toxine,
- au clonage du gène d'adénylates cyclases eucaryotes, en utilisant des clones contenant soit un gène de calmoduline, soit un gène de la sous-unité G activatrice de l'adénylate cyclase, soit un oncogène de type Ras, et à la production de ces cyclases,
- au clonage des gènes dont les produits sont modulés par la calmoduline,

- au clonage des gènes dont les produits sont modulés par la calmoduline,
- à la production de protéines oligomériques au moyen de couples de vecteurs par exemple immunoglobulines ou hormones,
- au clonage de gènes codant pour des récepteurs, activateurs d'enzymes repérables ou l'opposé, et à la production de ces protéines,
- au clonage de gènes codant pour des enzymes toxiques (par exemple protéases/antiprotéases) et à la production de ces enzymes. Dans ce cas, le clonage a lieu dans des conditions de production de l'inhibiteur (dans un système traditionnel par exemple) et la révélation des clones intéressants se fait après inhibition de la synthèse de l'inhibiteur par apparition de la toxicité,
- au clonage de gènes codant pour des inhibiteurs d'activités repérables, dont les gènes sont déjà connus, et à la production de ces inhibiteurs,
- à la production de protéines excrétées, nécessitant la présence d'une protéine auxiliaire pour l'excrétion.

## Références bibliographiques

(1) De Reuse et al, FEMS Microbiology Letters 37 (1986) 193-197

(2) Maniatis et al, Cold Spring harbor N.Y., p. 352-355, 1982

(3) Miller, 1972, Molecular Genetics, Cold Spring Harbor, N.Y.

(4) Roberts et al, 1987, Biochemistry, 1985, 24: 5090-5098

(5) Struhl et al, 1976, Proc.Natl Acad Sci USA 73: 1471-1475

(6) Kieny et al, 1983, Gene 26:91-99

(7) Sanger et al, 1977, Proc Natl Acad Sci USA 74: 5463-5467

(8) Caput et al, 1986, PNAS, vol. 83, p. 1670-74

**Revendications**

1. Procédé de clonage et d'expression de gènes codant pour des protéines ayant une parenté fonctionnelle caractérisé en ce que :
- on transforme les cellules d'une souche de microorganisme réceptrice, c'est-à-dire capable d'accueillir de l'ADN étranger et de l'exprimer, en introduisant des fragments d'ADN constitués par, ou comprenant, au moins deux gènes étrangers, à savoir au moins un gène indicateur et au moins un gène à révéler, ces gènes étant interchangeables, le cas échéant, en tant que gènes indicateurs et gènes à révéler, et capables d'exprimer dans les cellules réceptrices des protéines constituant des édifices fonctionnels organisés, le gène indicateur étant capable d'exprimer une protéine permettant de détecter l'activité biologique de la protéine exprimée par le gène à révéler, cette activité biologique résultant de l'interaction entre les protéines,

- on détecte l'activité biologique de la protéine recherchée par mise en oeuvre par exemple d'un moyen physico-chimique, biochimique, microbiologique ou radioimmunologique, et
- on isole, le cas échéant, le gène codant pour cette protéine en mettant en jeu une propriété caractéristique du gène.

2. Procédé selon la revendication 1, caractérisé en ce que la souche réceptrice est une souche bactérienne, une levure ou une cellule eucaryote.

3. Procédé selon la revendication 2, caractérisé en ce que la cellule réceptrice bactérienne présente un caractère restriction moins.

4. Procédé selon l'une quelconque des revendications 1 à 3, caractérisé en ce que les gènes étrangers introduits sont impliqués dans des systèmes homologues.

5. Procédé selon l'une quelconque des revendications 1 à 3, caractérisé en ce que les gènes étrangers introduits sont impliqués dans des systèmes hétérologues.

6. Procédé selon l'une quelconque des revendications 1 à 5, caractérisé en ce qu'on effectue un clonage successif des gènes codant pour les diverses unités de l'édifice multiprotéique en utilisant de préférence des réplicons différents portant les différents gènes.

7. Procédé selon l'une quelconque des revendications 1 à 5, caractérisé en ce qu'on utilise un seul type de réplicon.

8. Procédé de clonage du gène codant pour l'adénylate cyclase de B.pertussis, caractérisé en ce qu'il comprend :
- la transformation d'une cellule de microorganisme, en particulier d'une cellule bactérienne, à l'aide de deux réplicons A et B, différents l'un de l'autre, le réplicon A portant le gène codant pour la calmoduline et le réplicon B des fragments de l'ADN total de B.pertussis.
- le criblage des fragments d'ADN, en détectant par un test coloré l'activité biologique de l'AMP cyclique produite par l'adénylate cyclase codée par l'un des fragments d'ADN, activée en présence de calmoduline;
- l'isolement du gène codant pour l'adénylate cyclase.

9. Application du procédé selon la revendication 8 à la production d'adénylate cyclase.

10. Séquence de nucléotides codant pour au moins une partie de l'adénylate cyclase, caractérisée par sa capacité d'hybridation avec un gène capable d'exprimer une protéine à activité adénylate cyclase.

11. Séquence de nucléotides, caractérisée en ce qu'elle est capable de s'hybrider avec une sonde formée à partir de la séquence selon la figure 2a à 2f.

12. Séquence de nucléotides, caractérisée en ce qu'elle code pour la séquence d'acides aminés représentée sur la figure 2a à 2f.

13. Protéine à activité adénylate cyclase, caractérisée en ce qu'elle est capable de former un complexe immunologique avec des anticorps dirigés contre des protéines à activité adénylate cyclase.

14. Protéine selon la revendication 13, caractérisée en ce qu'elle comprend la séquence d'acides aminés représentée sur la figure 2a à 2f.

15. Procédé de clonage du gène codant pour la calmoduline, caractérisé en ce qu'il comprend :
- la transformation d'une cellule de microorganisme, en particulier d'une cellule bactérienne, à l'aide de deux réplicons A et B, différents l'un de l'autre, le réplicon A1 portant des fragments d'ADNc d'une banque d'ADNc de cerveau et le réplicon B1, le gène codant pour l'adénylate cyclase de B.pertussis.
- le criblage des fragments d'ADN, en détectant par un test coloré l'activité biologique de l'AMP cyclique produite par l'adénylate cyclase activée en présence de la calmoduline codée par l'un des fragments d'ADN;
- l'isolement du gène codant pour l'adénylate cyclase.

16. Produits tels qu'obtenus par mise en oeuvre du procédé selon l'une quelconque des revendications 1 à 7.

pDIA 7

E    VB V    VE Sa Sm    V    V B    E

Tet'    CYA

⊢—⊣  1000  pB

B  =  Bam H1
E  =  Ecr  R1
V  =  Eco  RⅤ
Sa =  Sac  1
Sm =  Sma 1

FIG.1

CGATCATTCGGCATGTACGGTCCAGCTGCGCGCGAGCGGCGGCCGCGTCCAGCGCGCGGC

CTCGGTACTCCTTGACGCGCGCGGTGTCGCCGCCGCGCCGAACGCGCAGCGAACGGCCCA

      -900

CGCTGTCGGGGTGCCGTTCGGCCAGCGCGCGGCGCAGCGCACGATTGTCGTCGCGCGAGA

                                                          -800

ATGGCGCGATCCAGTCGATGATCCACAGTCGGTCGCCGCAGTTCCAGGCATTCCCGCCCA

GCGACGAGGGCGCCATGACATAGGAGAGTTCGGTGTCGGCGTCCATTAGGGCCCAGCTGC

                                -700

AGTATGCAACCGGCACGTCATTGCATCGCAGCAGAATGTATTGGCCCAGTTGAATCGGCG

CGAGCGCTGTTGCGTGCGAGCAGATGCACCGGCCAGTCGCGGTGCATGGGAGAGTTCATC

      -600

CACAGCCAGGCAATATTGCCCAGTGCCGCGAAGTCGTCGGTGGGATTGAGGAGGGAGGGC

                                                -500

GCTTGGGCGGACGGAAGCATGACATCGGTGCATGGTGGAGCGGGGGGCATATTCCGTGTT

GGGTGCGCGCATGGCAAGCCGCCGGCGCATCATGGTTGCGCCGGAATGGCTTTTCTTACA

                                -400

TGTTTCCAGGATATGTCCGTATTTCGGGCGATGCCTCGGTCGCGGCGCCTGCTTTTGTCG

AACATGTGCAATGTTGTTGTCGCGATCGCGTTGGCGCTTGCTCGCTTATTTATCTCCCTT

      -300

GAAGCCTTGTTCTTCTTTTCATTAGAAAGAAATATGCGCTTTGTGTTTAGGATGATTTTC

-200

CTGTCCGAGTAGGGTGGATCCAAATTTTCCGGATTGGTGGGAATTTGTGCATTTTCACTG

CGAATGTTGGAATAATTTCGCCCATCGTCATACGACATGCTGGATGTTTGGTTCTTGCAG

-100

AAGGATGAGGTTCTGAGCGCTACACACCGGTTGCGTCGGTGCGAATCCGTTCAATCGACT

MetGlnGlnSerHisGlnAlaGlyTyrAlaAsnAlaAlaAsp  14

ACTTATCGACAGATCCACATGCAGCAATCGCATCAGGCTGGTTACGCAAACGCCGCCGAC

0

ArgGluSerGlyIleProAlaAlaValLeuAspGlyIleLysAlaValAlaLysGluLys  34

CGGGAGTCTGGCATCCCCGCAGCCGTACTCGATGGCATCAAGGCCGTGGCGAAGGAAAAA

100

AsnAlaThrLeuMetPheArgLeuValAsnProHisSerThrSerLeuIleAlaGluGly  54

AACGCCACATTGATGTTCCGCCTGGTCAACCCCCATTCCACCAGCCTGATTGCCGAAGGG

ValAlaThrLysGlyLeuGlyValHisAlaLysSerSerAspTrpGlyLeuGlnAlaGly  74

GTGGCCACCAAAGGATTGGGCGTGCACGCCAAGTCGTCCGATTGGGGGTTGCAGGCGGGC

200

TyrIleProValAsnProAsnLeuSerLysLeuPheGlyArgAlaProGluValIleAla  94

TACATTCCCGTCAACCCGAATCTTTCCAAACTGTTCGGCCGTGCGCCCGAGGTGATCGCG

```
ArgAlaAspAsnAspValAsnSerSerLeuAlaHisGlyHisThrAlaValAspLeuThr     114
CGGGCCGACAACGACGTCAACAGCAGCCTGGCGCATGGCCATACCGCGGTCGACCTGACG
              .      300        .        .         .          .

LeuSerLysGluArgLeuAspTyrLeuArgGlnAlaGlyLeuValThrGlyMetAlaAsp    134
CTGTCGAAAGAGCGGCTTGACTATCTGCGGCAAGCGGGCCTGGTCACCGGCATGGCCGAT
        .       .         .         .          .     400

GlyValValAlaSerAsnHisAlaGlyTyrGluGlnPheGluPheArgValLysGluThr    154
GGCGTGGTCGCGAGCAACCACGCAGGCTACGAGCAGTTCGAGTTTCGCGTGAAGGAAACC
        .       .         .        .      .    .          .

SerAspGlyArgTyrAlaValGlnTyrArgArgLysGlyGlyAspAspPheGluAlaVal    174
TCGGACGGGCGCTATGCCGTGCAGTATCGCCGCAAGGGCGGCGACGATTTCGAGGCGGTC
        .       .         .    500        .          .

LysValIleGlyAsnAlaAlaGlyIleProLeuThrAlaAspIleAspMetPheAlaIle    194
AAGGTGATCGGCAATGCCGCCGGTATTCCACTGACGGCGGATATCGACATGTTCGCCATT
        .       .         .         .        .           .

MetProHisLeuSerAsnPheArgAspSerAlaArgSerSerValThrSerGlyAspSer    214
ATGCCGCATCTGTCCAACTTCCGCGACTCGGCGCGCAGTTCGGTGACCAGCGGCGATTCG
        .      600        .         .        .          .

ValThrAspTyrLeuAlaArgThrArgArgAlaAlaSerGluAlaThrGlyGlyLeuAsp    234
GTGACCGATTACCTGGCGCGCACGCGGCGGGCCGCCAGCGAGGCCACGGGCGGCCTGGAT
        .       .         .         .      700

ArgGluArgIleAspLeuLeuTrpLysIleAlaArgAlaGlyAlaArgSerAlaValGly    254
CGCGAACGCATCGACTTGTTGTGGAAAATCGCTCGCGCCGGCGCCCGTTCCGCAGTGGGC
        .       .         .         .          .
```

2d    0301954

ThrGluAlaArgArgGlnPheArgTyrAspGlyAspMetAsnIleGlyValIleThrAsp    274
ACCGAGGCGCGTCGCCAGTTCCGCTACGACGGCGACATGAATATCGGCGTGATCACCGAT
.        .        .        800        .        .

PheGluLeuGluValArgAsnAlaLeuAsnArgArgAlaHisAlaValGlyAlaGlnAsp    294
TTCGAGCTGGAAGTGCGCAATGCGCTGAACAGGCGGGCGCACGCCGTCGGCGCGCAGGAC
.        .        .        .        .        .

ValValGlnHisGlyThrGluGlnAsnAsnProPheProGluAlaAspGluLysIlePhe    314
GTGGTCCAGCATGGCACTGAGCAGAACAATCCTTTCCCGGAGGCAGATGAGAAGATTTTC
.        900        .        .        .        .

ValValSerAlaThrGlyGluSerGlnMetLeuThrArgGlyGlnLeuLysGluTyrIle    334
GTCGTATCGGCCACCGGTGAAAGCCAGATGCTCACGCGCGGGCAACTGAAGGAATACATT
.        .        .        .        .        1000

GlyGlnGlnArgGlyGluGlyTyrValPheTyrGluAsnArgAlaTyrGlyValAlaGly    354
GGCCAGCAGCGCGGCGAGGGCTATGTCTTCTACGAGAACCGTGCATACGGCGTGGCGGGG
.        .        .        .        .        .

LysSerLeuPheAspAspGlyLeuGlyAlaAlaProGlyValProSerGlyArgSerLys    374
AAAAGCCTGTTCGACGATGGGCTGGGAGCCGCGCCCGGCGTGCCGAGCGGACGTTCGAAG
.        .        .        1100        .        .

PheSerProAspValLeuGluThrValProAlaSerProGlyLeuArgArgProSerLeu    394
TTCTCGCCGGATGTACTGGAAACGGTGCCGGCGTCACCCGGATTGCGGCGGCCGTCGCTG
.        .        .        .        .        .

GlyAlaValGluArgGlnAspSerGlyTyrAspSerLeuAspGlyValGlySerArgSer    414
GGCGCAGTGGAACGCCAGGATTCCGGCTATGACAGCCTTGATGGGGTGGGATCGCGATCG
.        1200        .        .        .        .

PheSerLeuGlyGluValSerAspMetAlaAlaValGluAlaAlaGluLeuGluMetThr

TTCTCGTTGGGCGAGGTGTCCGACATGGCCGCCGTGGAAGCGGCGGAACTGGAAATGACC

.     .     .     .     .     **1300**

ArgGlnValLeuHisAlaGlyAlaArgGlnAspAspAlaGluProGlyValSerGlyAla

CGGCAAGTCTTGCACGCCGGGGCGCGGCAGGACGATGCCGAGCCGGGCGTGAGCGGTGCG

.     .     .     .     .     .

SerAlaHisTrpGlyGlnArgAlaLeuGlnGlyAlaGlnAlaValAlaAlaAlaGlnArg

TCGGCGCACTGGGGGCAGCGGGCGCTGCAGGGCGCCCAGGCGGTGGCGGCGGCGCAGCGG

.     .     .     **1400**     .     .

LeuValHisAlaIleAlaLeuMetThrGlnPheGlyArgAlaGlySerThrAsnThrPro

CTGGTTCATGCCATTGCCCTGATGACGCAATTCGGCCGGGCCGGTTCCACCAACACGCCG

.     .     .     .     .     .

GlnGluAlaAlaSerLeuSerAlaAlaValPheGlyLeuGlyGluAlaSerSerAlaVal

CAGGAAGCGGCCTCGTTGTCGGCGGCCGTGTTCGGCTTGGGCGAGGCCAGCAGCGCCGTG

.     **1500**     .     .     .     .

AlaGluThrValSerGlyPhePheArgGlySerSerArgTrpAlaGlyGlyPheGlyVal

GCCGAAACCGTGAGCGGTTTTTTCCGCGGGTCTTCGCGCTGGGCCGGCGGTTTCGGCGTG

.     .     .     .     .     **1600**

AlaGlyGlyAlaMetAlaLeuGlyGlyGlyIleAlaAlaAlaValGlyAlaGlyMetSer

GCTGGCGGCGCGATGGCGCTGGGAGGCGGCATCGCCGCGGCCGTTGGCGCCGGGATGTCG

.     .     .     .     .     .

LeuThrAspAspAlaProAlaGlyGlnLysAlaAlaAlaGlyAlaGluIleAlaLeuGln

TTGACCGATGACGCGCCGGCCGGACAGAAGGCCGCCGCCGGCGCCGAGATCGCGCTGCAG

.     .     .     **1700**     .     .

```
LeuThrGlyGlyThrValGluLeuAlaSerSerIleAlaLeuAlaLeuAlaAlaAlaArg      594
TTGACAGGTGGAACGGTCGAGCTGGCTTCTTCCATCGCGTTGGCGCTGGCCGCGGCGCGC
      .           .           .           .           .           .

GlyValThrSerGlyLeuGlnValAlaGlyAlaSerAlaGlyAlaAlaAlaGlyAlaLeu      614
GGCGTGACCAGCGGCTTGCAGGTGGCCGGGGCGTCGGCCGGGGCGGCTGCCGGCGCATTG
      .       1800            .           .           .           .

AlaAlaAlaLeuSerProMetGluIleTyrGlyLeuValGlnGlnSerHisTyrAlaAsp     634
GCCGCGGCGCTCAGTCCCATGGAGATCTACGGCCTGGTGCAGCAATCGCACTATGCGGAT
      .           .           .           .           .       1900

GlnLeuAspLysLeuAlaGlnGluSerSerAlaTyrGlyTyrGluGlyAspAlaLeuLeu     654
CAGCTGGACAAGCTGGCGCAGGAATCGAGCGCATACGGTTACGAGGGCGACGCCTTGCTG
      .           .           .           .           .           .

AlaGlnLeuTyrArgAspLysThrAlaAlaGluGlyAlaValAlaGlyValSerAlaVal     674
GCCCAGCTGTATCGCGACAAGACGGCCGCCGAGGGCGCCGTCGCCGGCGTCTCCGCCGTC
      .           .           .       2000            .           .

LeuSerThrValGlyAlaAlaValSerIleAlaAlaAlaAlaSerValValGlyAlaPro     694
CTGAGCACGGTGGGGGCGGCGGTGTCGATCGCCGCGGCGGCCAGCGTGGTAGGGGCCCCG
      .           .           .           .           .           .

ValAlaValValThrSerLeuLeuThrGlyAlaLeuAsnGlyIleLeuArgGlyValGln     714
GTGGCGGTGGTCACTTCCTTGCTGACCGGGGCTCTCAACGGCATCCTGCGCGGCGTGCAG
      .       2100            .           .           .           .

GlnProIleIleGluLysLeuAlaAsnAspTyrAlaArgLysIleAspGluLeuGlyGly     734
CAGCCCATCATCGAAAAGCTGGCCAACGATTACGCTCGCAAGATCGACGAGCTGGGCGGG
      .           .           .           .           .       2200
```

ProGlnAlaTyrPheGluLysAsnLeuGlnAlaArgHisGluGlnLeuAlaAsnSerAsp 754
CCGCAAGCGTACTTCGAGAAAAACCTGCAGGCGCGTCACGAACAACTGGCCAATTCGGAC

   .       .       .       .       .       .

GlyLeuArgLysMetLeuAlaAspLeuGlnAlaGlyTrpAsnAlaSerSerValIleGly 774
GGCCTACGGAAAATGCTGGCCGACCTGCAGGCCGGTTGGAACGCCAGCAGCGTGATCGGG

   .       .       .    2300       .       .

ValGlnThrThrGluIleSerLysSerAlaLeuGluLeuAlaAlaIleThrGlyAsnAla 794
GTGCAGACGACAGAGATCTCCAAGTCGGCGCTCGAACTGGCCGCCATTACCGGCAACGCG

       .       .       .       .       .       .

AspAsnLeuLysSerValAspValPheValAspArgPheValGlnGlyGluArgValAla 814
GACAACCTGAAATCCGTCGACGTGTTCGTGGACCGCTTCGTCCAGGGCGAGCGGGTGGCC

   .    2400       .       .       .       .

GlyGlnProValValLeuAspValAlaAlaGlyGlyIleAspIleAlaSerArgLysGly 834
GGCCAGCCGGTGGTCCTCGACGTCGCCGCCGGCGGCATCGATATCGCCAGCCGCAAGGGC

   .       .       .       .       .    2500

GluArgProAlaLeuThrPheIleThrProLeuAlaAlaProGlyGluGluGlnArgArg 854
GAGCGGCCGGCGCTGACGTTCATCACGCCGCTGGCCGCGCCAGGAGAAGAGCAGCGCCGG

   .       .       .       .       .       .

ArgThrLysThrGlyLysSerGluPheThrThrPheValGluIleValGlyLysGlnAsp 874
CGCACGAAAACGGGCAAGAGCGAATTCACCACATTCGTCGAGATCGTGGGCAAGCAGGAC

   .       .       .    2600       .       .

ArgTrpArgIleArgAspGlyAlaAlaAspThrThrIleAspLeuAlaLysValValSer 894
CGCTGGCGCATCCGGGACGGCGCGGCCGACACCACCATCGATCTGGCCAAGGTGGTGTCG

   .       .       .       .       .       .

0301954

GlnLeuValAspAlaAsnGlyValLeuLysHisSerIleLysLeuAspValIleGlyGly    914

CAACTGGTCGACGCCAATGGCGTGCTCAAGCACAGCATCAAACTGGATGTGATCGGCGGA

. 2700 . . . .

AspGlyAspAspValValLeuAlaAsnAlaSerArgIleHisTyrAspGlyGlyAlaGly    934

GATGGCGATGACGTCGTGCTTGCCAATGCTTCGCGCATCCATTATGACGGCGGCGCGGGC

. . . . . 2800

ThrAsnThrValSerTyrAlaAlaLeuGlyArgGlnAspSerIleThrValSerAlaAsp    954

ACCAACACGGTCAGCTATGCCGCCCTGGGTCGACAGGATTCCATTACCGTGTCCGCCGAC

. . . . . .

GlyGluArgPheAsnValArgLysGlnLeuAsnAsnAlaAsnValTyrArgGluGlyVal    974

GGGGAACGTTTCAACGTGCGCAAGCAGTTGAACAACGCCAACGTGTATCGCGAAGGCGTG

. . . 2900 . .

AlaThrGlnThrThrAlaTyrGlyLysArgThrGluAsnValGlnTyrArgHisValGlu    994

GCTACCCAGACAACCGCCTACGGCAAGCGCACGGAGAATGTCCAATACCGCCATGTCGAG

. . . . . .

LeuAlaArgValGlyGlnValValGluValAspThrLeuGluHisValGlnHisIleIle    1014

CTGGCCCGTGTCGGGCAAGTGGTGGAGGTCGACACGCTCGAGCATGTGCAGCACATCATC

. 3000 . . . .

GlyGlyAlaGlyAsnAspSerIleThrGlyAsnAlaHisAspAsnPheLeuAlaGlyGly    1034

GGCGGGGCCGGCAACGATTCGATCACCGGCAATGCGCACGACAACTTCCTAGCCGGCGGG

. . . . . 3100

SerGlyAspAspArgLeuAspGlyGlyAlaGlyAsnAspThrLeuValGlyGlyGluGly    1054

TCGGGCGACGACAGGCTGGATGGCGGCGCCGGCAACGACACCCTGGTTGGCGGCGAGGGC

. . . . . .

```
GlnAsnThrValIleGlyGlyAlaGlyAspAspValPheLeuGlnAspLeuGlyValTrp      1074
CAAAACACGGTCATCGGCGGCGCCGGCGACGACGTATTCCTGCAGGACCTGGGGGTATGG
        .            .            .        3200          .            .

SerAsnGlnLeuAspGlyGlyAlaGlyValAspThrValLysTyrAsnValHisGlnPro      1094
AGCAACCAGCTCGATGGCGGCGCGGGCGTCGATACCGTGAAGTACAACGTGCACCAGCCT
        .            .            .            .            .            .

SerGluGluArgLeuGluArgMetGlyAspThrGlyIleHisAlaAspLeuGlnLysGly      1114
TCCGAGGAGCGCCTCGAACGCATGGGCGACACGGGCATCCATGCCGATCTTCAAAAGGGC
        .        3300            .            .            .            .

ThrValGluLysTrpProAlaLeuAsnLeuPheSerValAspHisValLysAsnIleGlu      1134
ACGGTCGAGAAGTGGCCGGCCCTGAACCTGTTCAGCGTCGACCATGTCAAGAATATCGAG
        .            .            .            .            .        3400

AsnLeuHisGlySerArgLeuAsnAspArgIleAlaGlyAspAspGlnAspAsnGluLeu      1154
AATCTGCACGGCTCCCGCCTAAACGACCGCATCGCCGGCGACGACCAGGACAACGAGCTC
        .            .            .            .            .            .

TrpGlyHisAspGlyAsnAspThrIleArgGlyArgGlyGlyAspAspIleLeuArgGly      1174
TGGGGCCACGATGGCAACGACACGATACGCGGCCGGGGCGGCGACGACATCCTGCGCGGC
        .            .            .        3500          .            .

GlyLeuGlyLeuAspThrLeuTyrGlyGluAspGlyAsnAspIlePheLeuGlnAspAsp      1194
GGCCTGGGCCTGGACACGCTGTATGGCGAGGACGGCAACGACATCTTCCTGCAGGACGAC
        .            .            .            .            .            .

GluThrValSerAspAspIleAspGlyGlyAlaGlyLeuAspThrValAspTyrSerAla      1214
GAGACCGTCAGCGATGACATCGACGGCGGCGCGGGGCTGGACACCGTCGACTACTCCGCC
        .        3600            .            .            .            .
```

0301954

MetIleHisProGlyArgIleValAlaProHisGluTyrGlyPheGlyIleGluAlaAsp
ATGATCCATCCAGGCAGGATCGTTGCGCCGCATGAATACGGCTTCGGGATCGAGGCGGAC
.          .          .          .          .          3700

LeuSerArgGluTrpValArgLysAlaSerAlaLeuGlyValAspTyrTyrAspAsnVal
CTGTCCAGGGAATGGGTGCGCAAGGCGTCCGCGCTGGGCGTGGACTATTACGATAATGTC
.          .          .          .          .          .

ArgAsnValGluAsnValIleGlyThrSerMetLysAspValLeuIleGlyAspAlaGln
CGCAATGTCGAAAACGTCATCGGTACGAGCATGAAGGATGTGCTCATCGGCGACGCGCAA
.          .          .          3800          .          .

AlaAsnThrLeuMetGlyGlnGlyGlyAspAspThrValArgGlyGlyAspGlyAspAsp
GCCAATACCCTGATGGGGCCAGGGCGGCGACGATACCGTGCGCGGCGGCGACGGCGATGAT
.          .          .          .          .          .

LeuLeuPheGlyGlyAspGlyAsnAspMetLeuTyrGlyAspAlaGlyAsnAspThrLeu
CTGCTGTTCGGCGGCGACGGCAACGACATGCTGTATGGCGACGCCGGCAACGACACCCTC
.          3900          .          .          .          .

TyrGlyGlyLeuGlyAspAspThrLeuGluGlyGlyAlaGlyAsnAspTrpPheGlyGln
TACGGGGGGGCTGGGCGACGATACCCTTGAAGGCGGCGCGGGCAACGATTGGTTCGGCCAG
.          .          .          .          .          4000

ThrGlnAlaArgGluHisAspValLeuArgGlyGlyAspGlyValAspThrValAspTyr
ACGCAGGCGCGCGAGCATGACGTGCTGCGCGGCGGAGATGGGGTGGATACCGTCGATTAC
.          .          .          .          .          .

SerGlnThrGlyAlaHisAlaGlyIleAlaAlaGlyArgIleGlyLeuGlyIleLeuAla
AGCCAGACCGGCGCGCATGCCGGCATTGCCGCGGGTCGCATCGGGCTGGGCATCCTGGCT
.          .          .          4100          .          .

AspLeuGlyAlaGlyArgValAspLysLeuGlyGluAlaGlySerSerAlaTyrAspThr

GACCTGGGCGCCGGCCGCGTCGACAAGCTGGGCGAGGCCGGCAGCAGCGCCTACGATACG

ValSerGlyIleGluAsnValValGlyThrGluLeuAlaAspArgIleThrGlyAspAla

GTTTCCGGTATCGAGAACGTGGTGGGCACGGAACTGGCCGACCGCATCACGGGCGATGCG

4200

GlnAlaAsnValLeuArgGlyAlaGlyGlyAlaAspValLeuAlaGlyGlyGluGlyAsp

CAGGCCAACGTGCTGCGCGGCGCGGGTGGCGCCGACGTGCTTGCGGGCGGCGAGGGCGAC

4300

AspValLeuLeuGlyGlyAspGlyAspAspGlnLeuSerGlyAspAlaGlyArgAspArg

GATGTGCTGCTGGGCGGCGACGGCGACGACCAGCTGTCGGGCGACGCCGGACGCGATCGC

LeuTyrGlyGluAlaGlyAspAspTrpPhePheGlnAspAlaAlaAsnAlaGlyAsnLeu

TTGTACGGCGAAGCCGGTGACGACTGGTTCTTCCAGGATGCCGCCAATGCCGGCAATCTG

4400

LeuAspGlyGlyAspGlyArgAspThrValAspPheSerGlyProGlyArgGlyLeuAsp

CTCGACGGCGGCGACGGCCGCGATACCGTGGATTTCAGCGGCCCGGGCCGGGGCCTCGAC

AlaGlyAlaLysGlyValPheLeuSerLeuGlyLysGlyPheAlaSerLeuMetAspGlu

GCCGGCGCAAAGGGCGTATTCCTGAGCTTGGGCAAGGGGTTCGCCAGCCTGATGGACGAA

4500

ProGluThrSerAsnValLeuArgAsnIleGluAsnAlaValGlySerAlaArgAspAsp

CCCGAAACCAGCAACGTGTTGCGCAATATCGAGAACGCCGTGGGCAGCGCGCGTGATGAC

4600

```
ValLeuIleGlyAspAlaGlyAlaAsnValLeuAsnGlyLeuAlaGlyAsnAspValLeu    1554
GTGCTGATCGGCGACGCAGGCGCCAACGTCCTCAATGCCTGGCGGGCAACGACGTGCTG
        .           .           .           .           .           .
SerGlyGlyAlaGlyAspAspValLeuLeuGlyAspGluGlySerAspLeuLeuSerGly    1574
TCCGGCGGCGCTGGCGACGATGTGCTGCTGGGCGACGAGGGCTCGGACCTGCTCAGCGGC
        .           .           .       4700        .           .
AspAlaGlyAsnAspAspLeuPheGlyGlyGlnGlyAspAspThrTyrLeuPheGlyVal    1594
GATGCGGGCAACGACGATCTGTTCGGCGGGCAGGGCGATGATACTTATCTGTTCGGGGTC
        .           .           .           .           .           .
GlyTyrGlyHisAspThrIleTyrGluSerGlyGlyGlyHisAspThrIleArgIleAsn   1614
GGGTACGGGCACGACACGATCTACGAATCGGGCGGCGGCCATGACACCATCCGCATCAAC
        .       4800        .           .           .           .
AlaGlyAlaAspGlnLeuTrpPheAlaArgGlnGlyAsnAspLeuGluIleArgIleLeu    1634
GCGGGGGCGGACCAGCTGTGGTTCGCGCGCCAGGGCAACGACCTGGAGATCCGCATTCTC
        .           .           .           .           .       4900
GlyThrAspAspAlaLeuThrValHisAspTrpTyrArgAspAlaAspHisArgValGlu    1654
GGCACCGACGATGCACTTACCGTGCACGACTGGTATCGCGACGCCGATCACCGGGTGGAA
        .           .           .           .           .           .
IleIleHisAlaAlaAsnGlnAlaValAspGlnAlaGlyIleGluLysLeuValGluAla    1674
ATCATCCATGCCGCCAACCAGGCGGTAGACCAGGCAGGCATCGAAAAGCTGGTCGAGGCA
        .           .           .       5000        .           .
MetAlaGlnTyrProAspProGlyAlaAlaAlaAlaAlaProProAlaAlaArgValPro    1694
ATGGCGCAGTATCCGGACCCCGGCGCGGCGGCGGCTGCCCCGCCGGCGGCGCGCGTGCCG
        . .         .           .           .           .           .
```

AspThrLeuMetGlnSerLeuAlaValAsnTrpArg***

GACACGCTGATGCAGTCCCTGGCTGTCAACTGGCGCTGAAGCGCCGTGAATCACGGCCCG          1706

            .          5100          .          .          .          .

CCTGCCTCGCGCGGCGGCGCCGTCTCTTTGCGTTCTTCTCCGAGGTATTTCCCATCATGA

            .          .          .          .          .          5200

CGTCGCCCGGGGCGCAATGCGCCAGCGTGCCCGATTCCGGGTTGCTCTGCCTGGTCATGC

            .          .          .    .          .          .          .


TGGCTCGCTATCACGGATTGGCAGCCGATCCCGAGCAGTTGCGGCATGAGTTCGCCGAG C

            .          .          .          5300          .          .


AGGCATTCTGTAGCGAAACGATACAGCCTGGCGGCGCGCCGGGTCGGCCTGAAAGTGCGG

            .          .          .          .          .          .


CGGCACCGACCCGCGCCGGCGCGGCTGCCACGCGCGCCGCTGCCGGCCATCGCGCTGGAC

            .          5400          .          .          .          .


CGGCAGGGCGGCTACTTTGTT